# EUROPEAN PATENT SPECIFICATION

(11) **EP 2 844 225 B1**
(45) Date of publication and mention of the grant of the patent: **09.01.2019**
(21) Application number: 13722004.2
(22) Date of filing: 03.05.2013
(51) Int. Cl.: A61K 9/08, A01N 43/90, A61K 47/10, A61K 47/14, A01N 25/02, A01N 25/04, A61K 31/7048

(54) **AVERMECTIN POUR-ON FORMULATION WITH REDUCED WITHDRAWAL TIME**
AVERMECTIN-AUFGIESSFORMULIERUNG MIT REDUZIERTER ABZUGSZEIT
FORMULATION À BASE D'AVERMECTINE EN PIPETTE CARACTÉRISÉE PAR UN DÉLAI D'ATTENTE RÉDUIT

(30) Priority: 03.05.2012 GB 201207767; 29.08.2012 GB 201215349
(43) Date of publication of application: 11.03.2015
(73) Proprietor: Norbrook Laboratories Limited, Newry, County Down BT35 6PU (GB)
(72) Inventor: REYNOLDS, Louise, County Down Northern Ireland BT35 6PU (GB); UMRETHIA, Manish, Newry County Down Northern Ireland BT35 6PU (GB); HILLAN, Andrew, Newry County Down Northern Ireland BT35 6PU (GB)
(74) Representative: HGF Limited
(86) International application number: PCT/GB2013/051159
(87) International publication number: WO 2013/164636

(56) References cited:
- WO-A1-01/20994
- WO-A1-2006/077429
- WO-A1-2009/070687
- WO-A1-2012/085160
- WO-A2-94/26113
- DANAHER M ET AL: "Review of methodology for the determination of macrocyclic lactone residues in biological matrices", JOURNAL OF CHROMATOGRAPHY B: BIOMEDICAL SCIENCES & APPLICATIONS, ELSEVIER, AMSTERDAM, NL, vol. 844, no. 2, 5 December 2006 (2006-12-05), pages 175-203, XP027982007, ISSN: 1570-0232 [retrieved on 2006-12-05]
- HAMZA AVCIOGLU ET AL: "Efficacy of eprinomectin againstin calves", TROPICAL ANIMAL HEALTH AND PRODUCTION, KLUWER ACADEMIC PUBLISHERS, DO, vol. 43, no. 2, 7 October 2010 (2010-10-07), pages 283-286, XP019870923, ISSN: 1573-7438, DOI: 10.1007/S11250-010-9699-7
- Merial Animal Health Limited: "Summary of Product Characteristics Eprinex", , 1 January 2010 (2010-01-01), XP55447924, Retrieved from the Internet: URL:http://www.hpra.ie/img/uploaded/swedoc uments/LicenseSPC_10857-003-001_0805201410 0947.pdf [retrieved on 2018-02-05]

## Description

The present invention relates to topical formulations comprising avermectins, such as eprinomectin, a method of their production and their use in the treatment and prevention of both ectoparasites and endoparasites of animals. The topical formulations also unexpectedly provide a reduced withdrawal period for edible tissues in meat producing animals.

The avermectin series of compounds are potent anthelmintic and antiparasitic agents against internal and external parasites. The natural product avermectins are disclosed in U.S. Pat. No. 4,310,519 to Albers-Schonberg et al., and the 22,23-dihydroavermectin compounds are disclosed in Chabala et al., U.S. Pat. No. 4,199,569. Administration of the avermectin compounds can occur orally, parenterally or topically.

Conventional formulations of current medicinal agents require a withdrawal period of a few weeks after application of the active compound before any tissue from meat producing animals can be provided for human consumption. EP0697814 discloses a composition that provides a zero milk withdrawal time for topically applied antiparasitic agents with regard to dairy animals, but does not disclose the meat withdrawal time for such compositions. International Patent Application WO 2009/070687 discloses transdermal formulations containing macrocyclic lactones (eg, Ivermectin and Eprinomectin) in combination with Glycol Ethers such as propylene glycol. Commercially available topical formulations of avermectins containing eprinomectin, butylated hydroxytoluene and propylene glycol octanoatedecanoate have meat withdrawal periods of 15 days (Eprinex® by Merial). The Summary of Product Characteristics for Eprinex®, which outlines the particulars of the drug product for health professionals is downloadable free of charge from the Irish Health Products Regulatory Authority.
A current problem associated with the formulations that were available prior to the present invention was a prolonged meat withdrawal period.

There is, therefore, a need to provide a formulation which can be effective against endoparasites and ectoparasites whilst providing a reduced withdrawal period for edible tissues in meat producing animals.

It has now been surprisingly found that a formulation comprising from about 0.001% w/v to about 15% w/v of at least one avermectin, based on the total weight of the composition, at least about 60% w/v of a solvent, based on the total weight of the composition, and at least one oil in an amount of up to about 30% w/v, based on the total weight of the composition, which is free of polymeric material, can produce a reduced withdrawal period for edible tissues in meat producing animals. The meat withdrawal time has been found to be less than about 15 days, even less than about 10 days, and even less than about five days, in meat producing animals, such as for example cows, pigs and sheep.

The composition is advantageous as it allows the avermectin to be absorbed steadily through the skin over a period of time. This avoids or reduces the risk of blood level peaks and troughs commonly encountered with oral dosage forms. Additionally, the composition is advantageous as it unexpectedly reduces the meat withdrawal time for meat producing animals.

Hence according to the present invention there is provided a topical anti-parasitic composition comprising:
(i) from about 0.001% w/v to about 15% w/v of at least one avermectin, based on the total volume of the composition, wherein the avermectin is eprinomectin;
(ii) at least about 60% w/v of isopropyl alcohol, based on the total volume of the composition; and
(iii) at least one oil in an amount of up to about 30% w/v, based on the total volume of the composition, wherein the oil comprises propylene glycol dicaprylate/caprate, carprylic acid/capric triglyceride or mixtures thereof, and wherein the composition is free of polymeric material.

The present invention further provides a process for the manufacture of the topical anti-parasitic composition as described herein comprising:
(i) combining the at least one oil with part of the solvent;
(ii) dissolving the avermectin in the oil and solvent mixture of step (i); and
(iii) adding as a final volume, the remainder of the solvent.

The present invention further provides a topical anti-parasitic composition as described herein for use in the treatment of the animal body by therapy.

The present invention further provides a topical anti-parasitic composition as described herein for use in the treatment and prevention of ectoparasites and endoparasites of animals, which comprises topically applying the composition to the skin of the animal.

The present invention further provides a topical anti-parasitic composition consisting of:
(i) from about 0.001% w/v to about 15% w/v of at least one avermectin, based on the total volume of the composition, wherein the avermectin is eprinomectin;
(ii) at least about 60% w/v of isopropyl alcohol, based on the total volume of the composition;
(iii) at least one oil in an amount of up to about 30% w/v, based on the total volume of the composition, wherein the oil comprises propylene glycol dicaprylate/caprate, carprylic acid/capric triglyceride or mixtures thereof, and wherein the composition is free of polymeric material; and
(iv) optionally at least one or more of the following additives: penetration enhancer, anti-oxidant, colouring agent, bittering agent, base, preservative, crystallisation inhibitor and additional active agent.

Finally, the present invention provides a topical anti-parasitic composition as substantially herein described with reference to the Examples.

The present invention will now be further described. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

As used herein, "meat withdrawal time," means the time elapsed from the last application of the composition to an animal until the concentration of the avermectin and/or its metabolites is equal to, or less than the maximum residue limits in edible tissues as determined by linear regression analysis with a one-sided upper tolerance limit of 95% or 99% with a 95% confidence, or when the concentration of the avermectin and/or its metabolites is less than the maximum residue limits in edible tissues, plus a safety span of 10-30% of the time elapsed to such point. The meat withdrawal time can be calculated using the statistical programs WT1.4 and WT1.1, written to calculate the withdrawal time for tissues by Heckman. The WT1.4 and WT1.1 programs comply with the committee for Veterinary Medicinal Products (CVMP) guideline EMEA/CVM/036/95, Approach Towards Harmonisation of Withdrawal Periods and have been distributed by the European Agency for the Evaluation of Medicinal Products (EMEA) in London. As used herein "maximum residue limit" means the concentration of the avermectin and/or its metabolites is equal to 50µg/kg in muscle tissue, equal to 250µg/kg in fat tissue, equal to 1500µg/kg in liver tissue and/or equal to 300µg/kg in kidney tissue.

In a preferred embodiment the concentration of avermectin in plasma of the animal is between about 17.9 and about 76.5 ng/ml after the last administration, by topical pour-on to a meat producing animal, of the avermectin at a dose rate of about 0.5 mg/kg.

As used herein, "meat producing animals", means any animal from which tissues are removed for human consumption and includes animals from the following groups: bovine, ovine, caprine and porcine. Specific examples include cows, sheep and pigs.

Avermectins are antiparasitic agents that are useful against a broad spectrum of endoparasites and ectoparasites in mammals. The basic avermectin compound may be isolated from the fermentation broth of the soil micro-organism, *Streptomyces avermitilis* (see U.S. Pat. No. 4,310,519).
The avermectin used in the compositions of the present invention is eprinomectin.

The avermectin may be effective against a range of parasites, including worms, insects and mites. In this regard, the avermectin may be one or more of an anthelmintic, insecticidal or miticidal agent. The avermectin may be effective against parasitic infections caused by external and/or internal parasites. Examples of external and internal parasites include Ostertagia spp., *Ostertagia lyrata* (adult), *Ostertagia ostertagi* (including inhibited *O*. *ostertagi*), *Cooperia* spp. (including inhibited *Cooperia* spp), *Cooperia oncophora, Cooperia pectinata, Cooperia punctata, Cooperia surnabada, Haemonchus placei*, *Trichostrongylus* spp., *Trichostrongylus axei*, *Trichostrongylus colubriformis*, *Bunostomum phlebotomum, Nematodirus helvetianus*, *Oesophagostomum* spp. (adult), *Oesophagostomum radiatum*, *Trichuris* spp (adult), *Dictyocaulus viviparous, Hypoderma bovis, H. lineatum, Chorioptes bovis*, *Sarcoptes scabiei*, *Damalinia bovis* (biting lice), *Linognathus vituli* (sucking lice), *Haematopinus eurysternus* (sucking lice), *Solenopotes capillatus* (sucking lice).

The amount of the avermectin present in the composition should be sufficient to provide a therapeutic amount of the active and a convenient dosage unit. Accordingly, the avermectin can be present in an amount of from about 0.001% w/v to about 15% w/v, preferably in an amount of from about 0.1% w/v to about 8% w/v, more preferably in an amount of from about 0.5% w/v to about 5 % w/v, and most preferably in an amount of about 0.5% w/v, based on the total volume of the composition.

The composition comprises a pharmaceutically or veterinarily acceptable solvent. The solvent is isopropyl alcohol.

The solvent can be present in an amount of at least about 60% w/v, based on the total volume of the composition. Preferably, the solvent is present in an amount of less than about 90% w/v, preferably in an amount of less than about 85% w/v, and most preferably in an amount of less than about 65% w/v, based on the total volume of the composition. The use of the solvent in these concentrations leads to the unexpected result of a reduced meat withdrawal time while still maintaining the effectiveness of the avermectin formulation.

The composition comprises at least one oil. The oil
comprises propylene glycol dicaprylate/caprate, caprylic/capric triglyceride or mixtures thereof.

The oil is present in an amount of up to about 30% w/v, preferably in an amount of from about 10% w/v to about 30% w/v, more preferably in an amount of from 20% w/v to about 30% w/v, even more preferably in an amount of from about 25% w/v to about 30% w/v and most preferably in an amount of about 30% w/v, based on the total volume of the composition.

The composition is free of polymeric material. As used herein "polymeric material" means a material consisting of repeating monomeric subunits, wherein the polymeric material has a molecular weight of at least about 150, as determined by Gel Permeation Chromatography (GPC). The polymeric material includes natural and synthetic polymeric materials.

Examples of polymeric materials excluded from the composition are polyvinyl pyrrolidone, polyvinyl alcohol, polyethylene glycol, methyl cellulose, ethyl cellulose, carboxy methyl cellulose, hydroxyethyl cellulose, hydrolyzed wheat protein, hydrolyzed animal protein, gelatin derivatives, collagen derivatives, acrylates/t-octylpropenamide copolymer and cationic quaternary amine salts.

The composition may also contain one or more of the following additives: penetration enhancer, anti-oxidant, colouring agent, bittering agent, base, preservative, crystallisation inhibitor and additional active agent.

In a preferred embodiment the composition also contains one or more penetration enhancer, anti-oxidant, colouring agent, bittering agent, base, preservative and additional active agent.

In another preferred embodiment the composition also contains one or more bittering agents, anti-oxidant, and penetration enhancer.

In another preferred embodiment the composition also contains one or more colouring agent, anti-oxidant and penetration enhancer.

In another preferred embodiment the composition also contains one or more anti-oxidant and penetration enhancer.

The penetration enhancer facilitates the delivery of the avermectin through the animal's skin. Any suitable penetration enhancer may be used. Examples of penetration enhancers include azones, fatty acids and alcohols, fatty acid esters, essential oils and their derivatives, terpenes, terpenoids, oxazolidinones, DMSO and mixtures of two or more thereof. Examples of suitable fatty acid esters include butyl stearate, C12-C15 alkyl benzoate, cetearyl ethylhexanoate, isopropyl myristate, cetyl palmiate, diisopropyl adipate, di-ethylhexyl adipate, caprylic/capric triglyceride, isocetyl stearate, isopropyl palmitate, lauryl lactate, myristyl myristate, ethylhexyl cocoate, ethylhexyl hydroxystearate, ethylhexyl palmitate, ethylhexyl pelargonate, ethylhexyl stearate, di-ethylhexyl succinate, propylene glycol dicaprylate, propylene glycol dicaprate, PPG-2 myristyl ether propionate, pentaerythrityl tetraisostearate, pentaerythrityl tetracaprylate, pentaerythrityl tetracaprate, cetyl esters, isotridecyl isononanoate, stearyl heptanoate, steryl caprylate, and/or mixtures of two or more thereof.

Preferably, the penetration enhancer is selected from at least one of fatty acids, fatty acid esters, essential oils and their derivatives and terpenoids. Mixtures of two or more such penetration enhancers may be used. In a preferred embodiment, the penetration enhancer is cetearyl ethylhexanoate and/or isopropyl myristate (available commercially as Crodamol CAP®). In another preferred embodiment, the penetration enhancer is an essential oil, such as menthol, or an essential oil derivative, such as p-menthan-3,8-diol. In another preferred embodiment, the penetration enhancer is a terpenoid, such as camphor.

The penetration enhancer can be present in an amount of at least about 1% w/v, preferably in an amount of at least about 2% w/v and most preferably in an amount of at least about 5% w/v, based on the total weight of the composition.

The composition can comprise a pharmaceutically or veterinarily acceptable anti-oxidant. Suitable anti-oxidants include alpha-tocopherol, gamma-tocopherol, delta-tocopherol, extracts of natural origin which are rich in tocopherol, L-ascorbic acid and its sodium or calcium salts, palmityl-DL-ascorbic acid, propyl gallate, octyl gallate, dodecyl gallate, sodium metabisulphate, butylhydroxyanisole (BHA), butylated hydroxytoluene (BHT) and mixtures of two or more thereof. Preferred anti-oxidants are butylated hydroxyanisole (BHA) and butylated hydroxytoluene (BHT). Most preferably, the anti-oxidant is butylated hydroxytoluene (BHT). Addition of anti-oxidants may be advantageous in extending the shelf-life of the compositions.

The anti-oxidant can be present in an amount of from about 0.0001% w/v to about 1% w/v, preferably in an amount of from about 0.001% w/v to about 0.5% w/v, more preferably in an amount of from about 0.01% w/v to 0.02% w/v, and most preferably in an amount of about 0.01% w/v, based on the total weight of the composition.

Colouring agents may also be incorporated in the composition to provide an appealing colour to the composition. The colouring agents should be selected to avoid chemical incompatabilities with the other ingredients in the composition. Examples of suitable colouring agents include Brilliant Blue Dye, FD & C Red #40, FD &C Blue #1 and FD & C Red #33.

Colouring agents can be present in an amount of from about 0 to about 0.1 % w/v, preferably in an amount of from about 0 to about 0.0001% w/v, more preferably in an amount of from about 0 to about 0.01% w/v, and most preferably in an amount of about 0.01% w/v, based on the total weight of the composition.

Bittering agents may also be incorporated into the composition. Bittering agents include denatonium benzoate, sucrose octaacetate, quercetin, brucine and quassin. Bittering agents can be present in an amount of from about 0 to about 0.1% w/v, preferably in an amount of about 0 to about 0.0001% w/v, more preferably in an amount of from about 0 to about 0.025% w/v, and most preferably in an amount of about 0.025% w/v, based on the total weight of the composition.

A base can also be incorporated into the composition. Examples of suitable bases include triethanolamine, potassium hydroxide, sodium hydroxide, barium hydroxide, calcium hydroxide, caesium hydroxide, strontium hydroxide, lithium hydroxide and rubidium hydroxide, and mixtures of two or more thereof. The base can be present in an amount of from about 0 to about 0.1% w/v, preferably in an amount of from about 0 to about 0.01% w/v, and more preferably in an amount of from about 0 to about 0.05% w/v, based on the total weight of the composition.

Preservatives can also be incorporated into the composition to protect the compositions from microbial contamination during use. The preservative is preferably selected from the group consisting of benzoic acid and the sodium or potassium salts thereof, scorbic acid and the sodium or potassium salts thereof, benzyl alcohol, methyl alcohol, phenolic compounds such as phenol, quaternary compounds such as benzalkonium chloride, methyl parahydoxybenzoate, ethyl parahydroxybenzoate, propyl parahydoxybenzoate, butyl parahydroxybenzoate, parabens such as methyl, ethyl, propyl and butyl and mixtures of two or more thereof. In a preferred embodiment the preservative is benzyl alcohol.

The preservative can be present in an amount of from about 0.02% w/v to about 5% w/v, preferably in an amount of from about 0.1% w/v to about 3% w/v, and most preferably in an amount of from about 0.2 % w/v to about 1% w/v, based on the total weight of the composition.

A crystallisation inhibitor can also be present. Crystallisation inhibitors are known in the art and include, but are not limited to, benzyl alcohol, mannitol, glycerol, sorbitol, lecithin; acrylic derivatives such as methacrylates, anionic surfactants such as alkaline stearates, in particular sodium, potassium or ammonium stearate; calcium stearate, triethanolamine stearate; sodium abietate; alkyl sulphates in particular sodium lauryl sulphate and sodium cetyl sulphate; sodium dodecylbenzenesulphonate, sodium dioctylsulphosuccinate; fatty acids, in particular those derived from coconut oil, cationic surfactants such as water-soluble quaternary ammonium salts of formula N⁺R'R"R"'R""Y⁻ in which the radicals R', R" and R"' are hydrocarbon radicals, optionally hydroxylated, and Y⁻ is an anion of a strong acid such as halide, sulphate and sulphonate anions; cetyltrimethylammonium bromide is among the cationic surfactants which can be used, amine salts of formula NR'R"R"' in which the radicals R', R" and R"' are optionally hydroxylated hydrocarbon radicals; octadecylamine hydrochloride is among the cationic surfactants which can be used, amphoteric surfactants such as lauryl-substituted betaine compounds, or preferably a mixture of at least two or more thereof of these crystallisation inhibitors.

The crystallisation inhibitor can be present in an amount of from about 0.001 % w/v to about 5 % w/v, preferably in an amount of from about 0.1% w/v to about 3% w/v, and most preferably in an amount of from about 0.2% w/v to about 1% w/v, based on the total weight of the composition.

The other actives may be selected from one or more of sulphonamides, such as clorsulon, salicylanilides, such as closantel or oxyclozadine, substituted phenols, such as nitroxynil and benzimidazoles, such as albendazole and triclabendazole.

Suitable amounts of the other-actives will depend on the active in question. Typically the other-actives may be present in an amount of from about 1% w/v to about 30% w/v, preferably in an amount of from about 1% w/v to about 15% w/v, based on the total weight of the composition.

Other actives include agents which with the composition of the present skin may be sprayed, squirted, or rubbed on to the skin. These include, for example conventional propellant gases required for spray cans, such as propane, butane, dimethyl ether, CO₂, or halogenated lower alkyl gases (for example, halogenated C₁-C₄ alkyls), and mixtures of two or more thereof.
In a preferred embodiment the composition comprises:
(i) about 0.5% w/v eprinomectin, based on the total weight of the composition;
(ii) about 30% w/v propylene glycol
   dicaprylate/caprate, based on the total weight of the composition; and
(iii) isopropyl alcohol quantity sufficient (q.s.) 100% w/v, based on the total weight of the composition, wherein the composition is free of polymeric material.

The compositions according to the invention are usually prepared by (i) combining the at least one oil with part of the solvent; (ii) dissolving the avermectin in the oil and solvent mixture of step (i); and (iii) adding as a final volume, the remainder of the solvent.

If optional constituents, such as bittering agent, penetration enhancer, antioxidant, crystallisation inhibitor, coluring agent, base, preservative and additional active agents are required, one or more of these can be added to the solvent and oil mixture and dissolved prior to the addition of the avermectin.

The composition of the present invention is a topical composition and so the composition may be in the form of a pour-on, spot-on or spray-on composition. Preferably, the composition is a pour-on composition.

The compositions according to the inventions are typically intended for meat producing animals, and are generally applied by deposition on the skin ("spot on" or "pour on" application). The composition may, for example, be applied at one, two or more points and is preferably localised between the animal's shoulders. The composition may also be applied in a continuous pouring action from a point between the animal's shoulders to a point near the base of the tail.

The composition of the present invention may be used to treat and prevent ectoparasites and endoparasites of animals, which comprises topically applying the composition to the skin of the animal. The larger the animal to be treated, the larger the dose volume of the composition to be applied.

The composition may be used to deliver the eprinomectin in an amount of from about 0.01 to about 10 mg per kg of body weight, preferably in an amount of from about 0.1 to about 5 mg per kg of body weight, more preferably in an amount of from about 0.5 to about 2 mg per kg of body weight. In one embodiment, the dose is about 0.5 mg per kg of body weight.

The composition of the present invention can also be used in the preparation of a medicament for the treatment and prevention of ectoparasites and endoparasites of animals.

The present invention will be further illustrated with reference to the following non-limiting Examples.

### Examples

### Example I - Compositions According to the Invention

The following compositions are exemplary of the compositions encompassed by the present invention.

| **Composition I** | |
|---|---|
| ***Ingredients*** | **% *w*/*v*** |
| Eprinomectin | 0.5 |
| cetearyl ethylhexanoate/isopropyl myristate | 5 |
| Propylene glycol dicaprylate/caprate | 30 |
| Denatonium Benzoate | 0.025 |
| Butylated hydroxytoluene | 0.01 |
| Isopropyl alcohol | q.s. 100 v/v |

| **Composition II** | |
|---|---|
| ***Ingredients*** | **% *w*/*v*** |
| Eprinomectin | 0.5 |
| cetearyl ethylhexanoate/isopropyl myristate | 5.0 |
| Caprylic/capric triglyceride | 30.0 |
| Brilliant Blue Dye | 0.01 |
| Butylated hydroxytoluene | 0.01 |
| Isopropyl alcohol | q.s. 100 v/v |

| **Composition III** | |
|---|---|
| ***Ingredients*** | **% *w*/*v*** |
| Eprinomectin | 0.5 |
| cetearyl ethylhexanoate/isopropyl myristate | 5.0 |
| Caprylic/capric triglyceride | 30.0 |
| Butylated hydroxytoluene | 0.01 |
| Isopropyl alcohol | q.s. 100 v/v |

| **Composition IV** | |
|---|---|
| ***Ingredients*** | **% w/v** |
| Eprinomectin | 0.5 |
| cetearyl ethylhexanoate/isopropyl myristate | 5.0 |
| Propylene glycol dicaprylate/caprate | 30.0 |
| Butylated hydroxytoluene | 0.01 |
| Isopropyl alcohol | q.s. 100 v/v |

### Example II - Plasma Levels of Eprinomectin Following Administration of an Eprinomectin Composition

Twenty beef cattle of mixed gender and aged approximately between 10-20 months at the time of selection were used to determine the levels of eprinomectin in plasma following topical administration of the composition set forth in Table 1 below at a dose rate of 0.5mg/kg of eprinomectin. The animals were weighed within 24 hours before administration of the eprinomectin composition and each calculated dose was rounded up to the nearest graduation on a syringe thereby ensuring each animal received the minimum nominal dose rate of 0.5mg eprinomectin/kg bodyweight.

**Table 1: Eprinomectin Composition**

| ***Ingredients*** | **% *w*/*v*** |
|---|---|
| Eprinomectin | 0.5 |
| Crodamol CAP (cetearyl ethyl hexanoate and/or isopropyl myristate) | 5 |
| Propylene glycol dicaprylate/caprate | 30 |
| Denatonium Benzoate | 0.025 |
| Butylated hydroxy toluene | 0.01 |
| Isopropyl alcohol | q.s. 100 |

The composition was administered along the midline of the back in a narrow strip between the withers and the tailhead using 60 ml syringes graduated to 1 ml. Blood samples were taken from each animal for eprinomectin determination at the following times: pre-treatment, 24, 48, 72, 96, 120, 144, 168, 192, 216, 240, 312, 336, 360 and 408 hours after administration. Blood samples were collected from each animal by venipuncture from the jugular vein using suitably labelled 9ml heparinised vacutainers. Following collection, samples were placed on ice protected from light under black cover prior to centrifugation. After centrifugation at 4500 revolutions per minute (RPM) for 5 minutes, the plasma was subsampled into 3 amber cryovials (-1ml plasma/cryovial). Plasma samples were stored at <-20°C until assayed. All samples were assayed for the determination of eprinomectin content, measured as eprinomectin B 1 a (hereafter referred to as eprinomectin) by HPLC.

Eprinomectin was extracted from plasma samples by solvent extraction with ethyl acetate:n-hexane (25:75% v/v), followed by sample concentration under nitrogen, derivatisation with trifluoroacetic anhydride and reconstitution in mobile phase. Final determination was by HPLC with fluorescence detection (excitation A.=360nm, emission A.=470nm). Quantitation was by measurement of sample peak responses in comparison to an internal standard peak response. The peak response ratio was compared with a calibration (standard) line prepared from spiked bovine plasma of known concentration. Only calibration (standard) lines with a coefficient of determination (r2) value of >99% and with an accuracy of at least six points, including the lowest concentration (LOQ), on the line of ± 15% of nominal concentration were deemed acceptable for the calculation of sample concentrations. The limit of quantitation (LOQ) for the assay was 1ppb (1ng/ml).

Eprinomectin was readily absorbed with animals presenting with plasma levels above LOQ by the first post treatment timepoint (24hours). At this timepoint, mean plasma concentrations ± SD of 14.3 ± 5.09 ppb were recorded for animals treated with the composition. Eprinomectin plasma concentrations continued to rise with a mean Cmax obtained at 96 hours. By 240 hours post administration, the mean plasma concentrations had depleted to a mean of 15.0 ± 6.69 ppb. Thereafter, the mean plasma concentrations gradually declined and at 408 hours a mean value of 2.9±1.15ppb was recorded.

The findings associated with pharmacokinetic parameters for eprinomectin for all animals are summarised below:

| **Parameter** | **Mean (± SD)** |
|---|---|
| Cmax (ppb) | 45.5 ± 18.39 |
| AUC (ppb hours) | 7843.6 ± 2699.66 |

The plasma levels of eprinomectin (ng/ml) in the individual animals are set forth in Table 2 below:

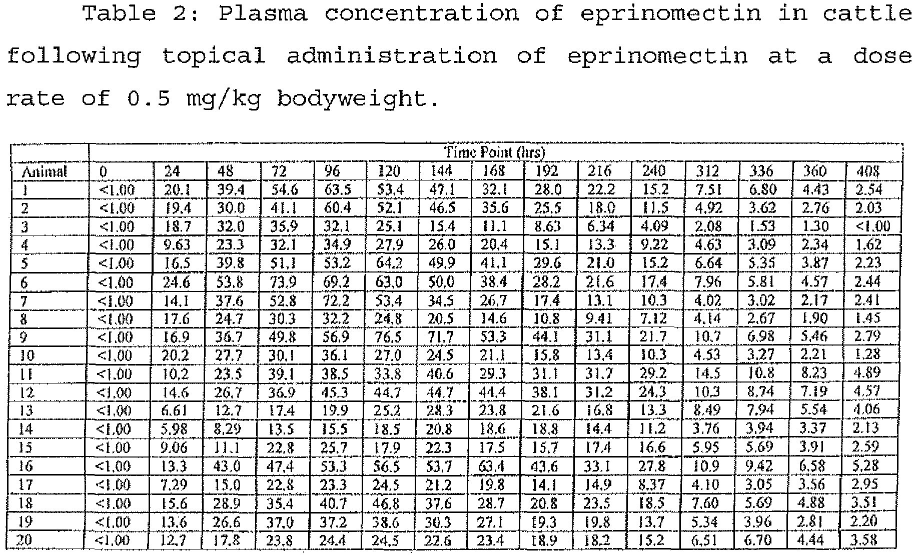

### Example III Tissue Residue Studies Conducted in Cattle following the topical administration of an eprinomectin composition

The maximum residue limits (MRL's) for eprinomectin have been set by the European Agency for the Evaluation of Medicinal Products, Veterinary Medicines Evaluation Unit(EEC Commission Regulation 2377/90) as follows:
Edible Tissues: 50pg/kg (muscle), 250µg/kg (fat), 1500µg/kg (liver), 300µg/kg (kidney).
Milk: 20µg/kg.

By comparing these values with data generated from residue studies it is possible to establish the appropriate withdrawal times for an eprinomectin composition.

A residue study was conducted in cattle to determine tissue levels of eprinomectin 5, 10, 15 and 20 days post administration of an eprinomectin composition, as described above in Example II. In this study, 4 groups of 4 cattle, aged approximately 11 to 19 months, comprising of 8 males and 8 females, weighing between 284 to 377kg, received the eprinomectin composition at a dose rate of 0.5mg eprinomectin per kg bodyweight administered topically on a single occasion. The animals were slaughtered by captive bolt stunning followed by exsanguination 5, 10, 15 and 20 days post treatment and the following tissues were harvested: muscle, liver, kidney and fat. All of the tissue samples weighed at least 100g. All tissue samples were stored whole at <-20°C prior to analysis for determination of the respective marker residues.

Eprinomectin was extracted from the tissue samples by solvent extraction with acetonitrile followed by sample concentration under nitrogen, isolation onto an Oasis HLB SPE cartridge, derivitisation with trifluoracetic anhydride and reconstitution in mobile phase. Final determination was by HPLC fluorescence detection. Quantitation was by measurement of the sample peak response in comparison to an ivermectin internal standard (IS) peak response. The peak response ratio was compared with a calibration (standard) line prepared from spiked tissue of known concentration. This method has a limit of quantitation (LOQ) of 0.025 ppm, (25 µg/kg).

The concentration of eprinomectin in each muscle (loin) sample was below the limit of quantification (LOQ = 25 µg/kg) for each animal at each slaughter timepoint. The concentration of eprinomectin in liver samples taken 5 days post administration ranged from 412 µg/kg to 727 µg/kg. For samples taken 10 days post administration the concentration ranged from 35.2 µg/kg to 377 µg/kg. Only two animals had levels above the LOQ for samples taken 15 and 20 days post administration, reporting 33.4 µg/kg and 47.2 µg/kg for animals slaughtered 15 days post administration and 26.9 µg/kg to 62 µg/kg for animals slaughtered 20 days post treatment.

The concentration of eprinomectin in kidney samples taken 5 days post administration ranged from 126 µg/kg to 228 µg/kg. Only two animals had levels above the LOQ for samples taken 10 days post administration, reporting 53.4 µg/kg and 92.3 µg/kg. Only one animal had levels above the LOQ for samples taken 15 days post administration reporting 30.5 µg/kg. No levels above the LOQ were reported for samples taken at the final slaughter timepoint 20 days post administration.

The concentration of eprinomectin in fat samples taken 5 days post administration ranged from 41.9 µg/kg to 101 µg/kg. No levels above the LOQ were reported for any animal when fat samples were taken 10, 15 and 20 days post administration. The results are presented in the tables below.

**Table 3. Mean levels of eprinomectin (µg/kg) in tissues of cattle 5 days post topical administration of eprinomectin composition at a dose rate of 0.5mg eprinomectin/kg bodyweight.**

| Animal | Tissue(µg/kg) | | | |
|---|---|---|---|---|
| | Muscle | Liver | Kidney | Fat |
| 1 | <25 | 541 | 228 | 43.7 |
| 2 | <25 | 727 | 165 | 101 |
| 3 | <25 | 571 | 168 | 93.6 |
| 4 | <25 | 412 | 126 | 41.9 |

**Table 4. Mean levels of eprinomectin (µg/kg) in tissues of cattle 10 days post topical administration of eprinomectin composition at a dose rate of 0.5mg eprinomectin/kg bodyweight.**

| Animal | Tissue(µg/kg) | | | |
|---|---|---|---|---|
| | Muscle | Liver | Kidney | Fat |
| 5 | <25 | 35.2 | <25 | <25 |
| 6 | <25 | 377 | 92.3 | <25 |
| 7 | <25 | 204 | 53.4 | <25 |
| 8 | <25 | 98.3 | <25 | <25 |

**Table 5. Mean levels of eprinomectin (µg/kg) in tissues of cattle 15 days post topical administration of eprinomectin composition at a dose rate of 0.5mg eprinomectin/kg bodyweight.**

| Animal | Tissue (µg/kg) | | | |
|---|---|---|---|---|
| | Muscle | Liver | Kidney | Fat |
| 9 | <25 | 47.2 | 30.5 | <25 |
| 10 | <25 | 33.4 | <25 | <25 |
| 11 | <25 | <25 | <25 | <25 |
| 12 | <25 | <25 | <25 | <25 |

**Table 6. Mean levels of eprinomectin (µg/kg) in tissues of cattle 20 days post topical administration of eprinomectin composition at a dose rate of 0.5mg eprinomectin/kg bodyweight.**

| Animal | Tissue (µg/kg) | | | |
|---|---|---|---|---|
| | Muscle | Liver | Kidney | Fat |
| 13 | <25 | 62 | <25 | <25 |
| 14 | <25 | 26.9 | <25 | <25 |
| 15 | <25 | <25 | <25 | <25 |
| 16 | <25 | <25 | <25 | <25 |

Using the results from Tables 3 to 6 and using the statistical programs WT1.1 and WT1.4 the meat withdrawal time for the composition tested is 9.58 days.

## Claims

1. A topical anti-parasitic composition comprising:
(i) from 0.001% w/v to 15% w/v of at least one avermectin, based on the total volume of the composition, wherein the at least one avermectin is eprinomectin;
(ii) at least 60% w/v of isopropyl alcohol solvent, based on the total volume of the composition; and
(iii)at least one oil in an amount of up to 30% w/v, based on the total volume of the composition, wherein the oil comprises propylene glycol dicaprylate/caprate, caprylic acid/capric triglyceride or mixtures thereof and wherein the composition is free of polymeric material.

2. The topical anti-parasitic composition of claim 1, wherein the avermectin is present in an amount of from 0.5% w/v to 5 % w/v, based on the total volume of the composition.

3. The topical anti-parasitic composition of any one of the preceding claims, wherein the oil is present in an amount of from 20% w/v to 30% w/v, based on the total volume of the composition.

4. The topical anti-parasitic composition of any one of the preceding claims further comprising an anti-oxidant.

5. The topical anti-parasitic composition of any one of the preceding claims, further comprising cetearyl ethylhexanoate and/or isopropyl myristate.

6. The topical anti-parasitic composition of any one of the preceding claims in the form of a pour-on formulation.

7. A process for the preparation of the topical anti-parasitic composition as defined in any one of the preceding claims comprising:
(i) combining the at least one oil with part of the solvent;
(ii) dissolving the avermectin in the oil and solvent mixture of step (i); and
(iii)adding as a final volume, the remainder of the solvent.

8. A composition as defined in any one of claims 1 to 6 for use in the treatment of the animal body by therapy.

9. A composition as defined in any one of claims 1 to 6 for use in the treatment and prevention of ectoparasites and endoparasites of animals, which comprises topically applying the composition to the skin of the animal.

## Patentansprüche

1. Topische antiparasitäre Zusammensetzung, die Folgendes beinhaltet:
(i) von 0,001 % w/v bis 15 % w/v von mindestens einem Avermectin, basierend auf dem Gesamtvolumen der Zusammensetzung, wobei das mindestens eine Avermectin Eprinomectin ist;
(ii) mindestens 60 % w/v von Isopropylalkohollösungsmittel, basierend auf dem Gesamtvolumen der Zusammensetzung; und
(iii) mindestens ein Öl in einer Menge bis 30 % w/v, basierend auf dem Gesamtvolumen der Zusammensetzung, wobei das Öl Propylenglycoldicaprylat/caprat, Caprylsäure/Caprintriglycerid oder Mischungen davon umfasst und wobei die Zusammensetzung frei von polymerem Material ist.

2. Topische antiparasitäre Zusammensetzung gemäß Anspruch 1, wobei das Avermectin in einer Menge von 0,5 % w/v bis 5 % w/v vorliegt, basierend auf dem Gesamtvolumen der Zusammensetzung.

3. Topische antiparasitäre Zusammensetzung gemäß einem der vorhergehenden Ansprüche, wobei das Öl in einer Menge von 20 % w/v bis 30 % w/v vorliegt, basierend auf dem Gesamtvolumen der Zusammensetzung.

4. Topische antiparasitäre Zusammensetzung gemäß einem der vorhergehenden Ansprüche, die ferner ein Antioxidans beinhaltet.

5. Topische antiparasitäre Zusammensetzung gemäß einem der vorhergehenden Ansprüche, die ferner Cetearylethylhexanoat und/oder Isopropylmyristat beinhaltet.

6. Topische antiparasitäre Zusammensetzung gemäß einem der vorhergehenden Ansprüche in Form einer Aufguss-Formulierung.

7. Verfahren für die Zubereitung der topischen antiparasitären Zusammensetzung, wie in einem der vorhergehenden Ansprüche definiert, der Folgendes beinhaltet:
(i) Kombinieren des mindestens einen Öls mit einem Teil des Lösungsmittels;
(ii) Auflösen des Avermectins in der Öl- und Lösungsmittelmischung von Schritt (i); und
(iii) Hinzufügen als endgültiges Volumen des Rests des Lösungsmittels.

8. Zusammensetzung, wie in einem der Ansprüche 1 bis 6 definiert, zur Verwendung bei der Behandlung des tierischen Körpers durch Therapie.

9. Zusammensetzung, wie in einem der Ansprüche 1 bis 6 definiert, zur Verwendung bei der Behandlung und Prävention von Ektoparasiten und Endoparasiten von Tieren, die topische Anwendung der Zusammensetzung auf der Haut des Tieres beinhaltet.

## Revendications

1. Composition antiparasitaire topique comprenant :
(i) de 0,001 % en poids/volume à 15 % en poids/volume d'au moins une avermectine, sur la base du volume total de la composition, dans laquelle au moins une avermectine est l'éprinomectine ;
(ii) au moins 60 % en poids/volume de solvant à l'isopropanol, sur la base du volume total de la composition ; et
(iii) au moins une huile en quantité jusqu'à 30 % en poids/volume, sur la base du volume total de la composition, l'huile comprenant du dicaprylate/caprate de propylène glycol, les triglycérides acide caprylique/caprique ou des mélanges de ceux-ci, et la composition étant exempte de polymère.

2. Composition antiparasitaire topique selon la revendication 1, dans laquelle l'avermectine est présente à raison de 0,5 % en poids/volume à 5 % en poids/volume, sur la base du volume total de la composition.

3. Composition antiparasitaire topique selon l'une quelconque des revendications précédentes, dans laquelle l'huile est présente à raison de 20 % en poids/volume à 30 % en poids/volume, sur la base du volume total de la composition.

4. Composition antiparasitaire topique selon l'une quelconque des revendications précédentes, comprenant en outre un antioxydant.

5. Composition antiparasitaire topique selon l'une quelconque des revendications précédentes, comprenant en outre de l'éthylhexanoate de cétéaryle et/ou du myristate d'isopropyle.

6. Composition antiparasitaire topique selon l'une quelconque des revendications précédentes sous la forme d'une formulation pour traitement cutané.

7. Procédé pour la préparation de la composition antiparasitaire telle que définie dans l'une quelconque des revendications précédentes, comprenant :
(i) la combinaison de la ou des huiles avec une partie du solvant ;
(ii) la dissolution de l'avermectine dans le mélange d'huile et de solvant de l'étape (i) ; et
(iii) l'adjonction du reste du solvant comme volume final.

8. Composition telle que définie dans l'une quelconque des revendications 1 à 6 pour utilisation dans le traitement du corps animal par thérapie.

9. Composition telle que définie dans l'une quelconque des revendications 1 à 6 pour utilisation dans le traitement et la prévention des ectoparasites et des endoparasites des animaux, qui comprend l'application topique de la composition sur la peau de l'animal.
